# EUROPÄISCHE PATENTSCHRIFT

(11) **EP 2 872 194 B1**
(45) Veröffentlichungstag und Bekanntmachung des Hinweises auf die Patenterteilung: **14.09.2016**
(21) Anmeldenummer: 13735021.1
(22) Anmeldetag: 10.07.2013
(51) Int. Cl.: A61M 25/00, A61M 39/22

(54) **BLASENKATHETER**
BLADDER CATHETER
SONDE URINAIRE

(30) Priorität: 13.07.2012 DE 202012006814 U
(43) Veröffentlichungstag der Anmeldung: 20.05.2015
(73) Patentinhaber: Rethink medical SL. Fundación Parque Cientifico Tecnológico de la Universidad de las Palmas de Gran Canaria, 35017 Las Palmas de Gran Canaria (ES)
(72) Erfinder: LUQUE GONZALÉS, Manuel, 29007 Malaga (ES)
(74) Vertreter: DREISS Patentanwälte PartG mbB
(86) Internationale Anmeldenummer: PCT/EP2013/064520
(87) Internationale Veröffentlichungsnummer: WO 2014/009389

(56) Entgegenhaltungen:
- WO-A1-2008/144622
- US-A- 5 460 606
- US-A- 5 476 434
- US-A1- 2005 148 999
- US-A1- 2006 095 019
- US-A1- 2006 212 024
- US-B1- 6 902 146

## Beschreibung

Die Erfindung betrifft einen Blasenkatheter mit einem Schlauch, wobei der Schlauch einen Einführabschnitt zum Einführen über eine Harnröhre in eine Harnblase aufweist und wobei der Schlauch einen Anschlussabschnitt zum Anschließen von Anschlusselementen aufweist, wobei durch den Einführabschnitt Fluid aus der Harnröhre am Anschlussabschnitt ablassbar ist wobei zwischen dem Einführabschnitt und dem Anschlussabschnitt ein verschließbares Ventil derart angeordnet ist, dass ein Austreten von Fluid aus dem Anschlussabschnitt bei verschlossenem Ventil verhindert werden kann.

Derartige Blasenkatheter sind aus dem Stand der Technik vielfach bekannt und aus dem medizinischen Bereich nicht mehr weg zu denken. Solche Blasenkatheter finden beispielsweise in Krankenhäusern Anwendung, bei Patienten, welche das Bett auf Grund einer Operation oder auf Grund Ihres Alters nicht mehr verlassen können. Auch bei anderen Störungen der Blasenentleerung werden derartige Blasenkatheter eingesetzt. Solche transurethralen Blasenkatheter, das heißt Blasenkatheter, welche über die Harnröhre in die Harnblase eingeführt werden, sind in unterschiedlichen Formen bekannt. So gibt es beispielsweise Einmalkatheter, welche nach der einmaligen Entleerung der Harnblase wieder aus der Harnröhre entfernt werden. Bei Dauerkathetern, welche in der Regel mindestens als ein 2-Wege-Katheter ausgebildet sind, ist ein Ballon an der Spitze des Blasenkatheters angeordnet, wobei der Blasenkatheter über einen Kanal zur Harnableitung und einen Kanal zum Füllen des Ballons bei eingeführtem Blasenkatheter verfügt. Durch den Ballon wird der Blasenkatheter dann in der Harnröhre dauerhaft befestigt.

Darüber hinaus sind aus dem Stand der Technik auch 3-Wege-Katheter, die sogenannten Spülkatheter, bekannt. Im Gegensatz zu einem 2-Wege-Katheter verfügen solche Blasenkatheter noch über einen zusätzlichen Kanal, durch welchen Spüllösungen in die Harnblase eingebracht werden können. Solche 3-Wege-Katheter kommen beispielsweise bei starken Blutungen in der Harnblase zum Einsatz, um ein Gerinnen von Blut in der Harnblase durch Spülen der Harnblase zu verhindern. Weitere Blasenkatheter sind aus der DE 20 2008 017 850 U1, der US 5,429,620 bekannt, der US 5 476 534 A, der US 2006/095019 A1, der US 2006/212024 A1, der US 6 902 146 B1, der WO 2008/144622 A1 und aus der US 2005/148999 A1 bekannt.

Diese aus dem Stand der Technik bekannten Blasenkatheter weisen jedoch einige Nachteile auf. Die bekannten Blasenkatheter müssen mit einem Urinbeutel zum Auffangen des aus dem Anschlussabschhitt austretenden Urins ausgestattet werden. Beim Wechsel eines solchen Urinbeutels, das heißt wenn der Urinbeutel voll ist, muss der Anschlussabschnitt über die Höhe der Harnröhre gehalten werden, da ansonsten Harn aus dem Anschlussabschnitt austreten kann. Gerade in einer Krankenhausumgebung stellt dies eine für die Patienten und für das Personal unzumutbare Beeinträchtigung dar. Darüber hinaus kann bei angeschlossenem Urinbeutel Harn jederzeit aus der Harnblase durch den Einführabschnitt am Anschlussabschnitt in den Urinbeutel abgelassen werden. Patienten welche über eine längere Zeit einen Blasenkatheter tragen, verlieren somit ihren natürlichen Blasenreflex. Nach Entfernen des Blasenkatheters kann dies zu Problemen beim natürlichen "Wasserlassen" der Patienten führen.

Die Erfindung stellt sich daher die Aufgabe, einen Blasenkatheter bereitzustellen, mit dem der Komfort für einen Patienten erhöht werden kann.

Diese Aufgabe wird mit einem Blasenkatheter mit den Merkmalen des Anspruchs 1 gelöst. Demgemäß ist vorgesehen, dass das Ventil als Schiebeventil ausgebildet ist und drei Schaltstellungen aufweist, wobei das Ventil in einer Verschlusslage geschlossen ist und wobei das Ventil in einer Mittelstellung luftdurchlässig ist und wobei das Ventil in einer Freigabelage geöffnet ist. Dies ist vorteilhaft, da dann, bei verschlossenem Ventil, ein voller Urinbeutel gewechselt werden kann, ohne dass Urin aus dem Anschlussabschnitt des Blasenkatheters austreten kann. Es ist somit nicht nur hygienischer, auch aus ökonomischen Gesichtspunkten ist ein solcher Blasenkatheter von Vorteil, da durch einen solchen Blasenkatheter in einem Krankenhaus die Entstehen von Kosten vermieden werden kann, da beispielsweise Hygienemaßnahmen, welche bislang notwendig waren, entfallen können. Mit einem Schiebeventil kann ein besonders sicheres Abdichten bei einer gleichzeitig sehr einfachen Bedienbarkeit ermöglicht werden. Ein Patient oder medizinisches Personal kann dann einfach und sicher Urin aus einer Harnblase ablassen beziehungsweise den Abfluss von Harn durch Schließen des Schiebeventils wieder unterbinden. Das Vorsehen von drei Schaltstellungen ist besonders vorteilhaft, da in der geschlossenen Mittelstellung ermöglicht wird, Luft aus der Blase eines Patienten zu entfernen, wobei ein Austreten von Urin verhindert werden kann.

Auch der Einsatz von Urinbeuteln kann mit einem solchen Blasenkatheter vermieden werden. Mit einem derartigen Blasenkätheter kann dann gewährleistet werden, dass ein Patient seinen Blasenreflex nicht verziert. Dieser bleibt vielmehr erhalten. Auf den Einsatz eines Urinbeutels kann verzichtet werden, da ein Patient, wenn er das Gefühl einer vollen Blase verspürt, eine Toilette aufsuchen kann, wobei der Patient dann seine Harnblase durch Öffnen des verschließbaren Ventils in eine Toilette entleeren kann. Gerade aus ergonomischen Gesichtspunkten kann ein solcher Blasenkatheter den Komfort für den Patienten enorm erhöhen. Ein Patient muss nicht mit einem Urinbeutel herumlaufen. Er kann vielmehr konventionelle, beispielsweise öffentliche Toiletten, benutzen. Der Blasenkatheter kann nach Entleerung der Harnblase in der Unterwäsche versteckt werden, ohne dass die Bekleidung nass wird.

Nachts kann dann, wie bei den aus dem Stand der Technik bekannten Blasenkathetern, ein Urinbeutel an den Anschlussabschnitt des Blasenkatheters zur Aufnahme von Urin angeschlossen werden.

Vorteilhafterweise kann ein solcher Blasenkatheter beziehungsweise der Schlauch aus Gummi und/oder Latex und/oder Polyvinylchlorid PVC und/oder Silikon hergestellt sein. Vorteilhafterweise weist die Oberfläche des Schlauches eine hydrophile Beschichtung zur Erhöhung der Gleitfähigkeit in die Harnröhre auf.

Weiterhin ist es vorteilhaft, wenn im Ventil ein luftdurchlässiger Filter- und/oder Membranabschnitt vorgesehen ist. Ein solcher Filter und/oder Membranabschnitt kann dabei vorteilhafterweise derart angeordnet sein, dass er bei Bewegen des Ventils in die Mittelstellung ein Austreten von Luft ermöglicht, wobei ein Austreten von Urin verhindert werden kann.

Besonders bevorzugt ist dabei, wenn der Filter- und/oder Membranabschnitt aus einem hydrophoben Acrylpolymer hergestellt ist.

Vorteilhafterweise weist der Blasenkatheter an seinem dem Anschlussabschnitt abgewandten Ende eine geschlossene Hohlspitze mit wenigstens zwei gegenüberliegenden Öffnungen nach der Nelaton-Bauweise auf. Es ist jedoch auch denkbar, dass der Blasenkatheter ein Foley-Katheter sein kann. Ein solcher Foley-Katheter hat eine Nelaton-Spitze und weist an seiner Spitze einen zusätzlichen Ballon zur Fixierung des Blasenkatheters in der Harnblase auf. Die Ausführung eines solchen Blasenkatheters als Nelaton-Katheter ist von Vorteil, da ein Nelaton-Katheter besonders leicht in eine Harnröhre eines Patienten eingeführt werden kann.

Besonders bevorzugt ist dabei, wenn der Blasenkatheter ein transurethraler Katheter ist. Ein transurethraler Blasenkatheter kann im Gegensatz zu einem suprapubischen Blasenkatheter durch die Harnröhre eines Patienten in die Harnblase eingeführt werden.

Eine weitere vorteilhafte Ausgestaltung des Blasenkatheters sieht vor, dass der Blasenkatheter ein Verweilkatheter ist. Dabei kann der Blasenkatheter insbesondere ein 2-Wege-Katheter sein, wobei durch einen zentralen Kanal Harn aus einer Harnröhre abgeleitet werden kann, wohingegen durch einen zweiten Kanal ein Ballon, welcher an einer Spitze des Blasenkatheters vorgesehen sein kann, mit Wasser gefüllt werden kann. Ein solcher Verweilkatheter kann dann in besonders vorteilhafter Weise selbsthaltend in einer Harnröhre angeordnet werden.

Eine weitere vorteilhafte Ausgestaltung des Blasenkatheters sieht vor, dass der Blasenkatheter ein Spülkatheter ist. Ein solcher Spülkatheter kann insbesondere ein 3-Wege-Katheter sein, bei dem im Gegensatz zu einem 2-Wege-Katheter durch einen zusätzlichen dritten Kanal eine Spüllösung in eine Harnblase eingebracht werden kann. Mit einem Spülkatheter kann zur Verhinderung von Blutgerinnung in der Harnblase eine Harnblase mit Spülflüssigkeit gespült werden.

Weitere Einzelheiten und vorteilhafte Ausgestaltungen der Erfindung sind der folgenden Beschreibung zu entnehmen, anhand derer die in den Figuren dargestellten Ausführungsformen der Erfindung näher beschrieben und erläutert sind. Es zeigen:
- Figur 1: eine Darstellung eines nicht erfindungsgemäßen Blasenkatheters in der Gesamtdarstellung;
- Figur 2: eine vergrößerte Darstellung der Spitze des Blasenkatheters gemäß Figur 1;
- Figur 3: eine Darstellung des Anschlussabschnitts des Blasenkatheters gemäß Figur 1;
- Figur 4a: ein Schiebeventil des Blasenkatheters gemäß Figur 1 in der Verschlusslage;
- Figur 4b: das Schiebeventil gemäß Figur 4a in der Freigabelage;
- Figur 5a: eine zweite Ausführungsform eines Schiebeventils gemäß Figur 1 in der Verschlusslage;
- Figur 5b: das Schiebeventil gemäß Figur 5a in einer Mittelstellung; und
- Figur 5c: das Schiebeventil gemäß Figur 5a in Freigabelage.

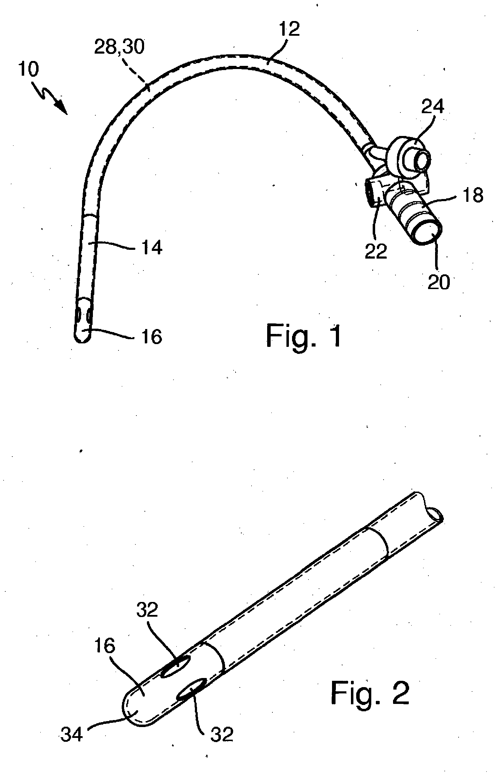

Figur 1 zeigt einen Blasenkatheter 10 mit einem Schlauch 12. Dieser Schlauch 12 weist einen Einführabschnitt 14 mit einer in der Figur 2 abgebildeten Spitze 16 auf. Dieser Einführabschnitt 14 kann zumindest abschnittsweise durch eine Harnröhre in eine Harnblase eines Patienten eingeführt werden. Der Schlauch 12 weist an seiner dem Einführabschnitt 14 beziehungsweise der Spitze 16 abgewandten Ende einen Anschlussabschnitt 18 auf. In diesen Anschlussabschnitt 18 beziehungsweise in die Öffnung 20 des Anschlussabschnitts 18 sind in den Figuren nicht dargestellte Anschlusselemente einführbar. Der Blasenkatheter 10 weist an seinem dem Anschlussabschnitt 18 zugewandten Ende ein als Schiebeventil ausgebildetes Ventil 22 auf. Dieses Ventil 22 ist derart zwischen dem Einführabschnitt 14 und dem Anschlussabschnitt 18 angeordnet, dass ein Austreten von Urin aus der Öffnung 20 beziehungsweise dem Anschlussabschnitt 18 bei geschlossenem Ventil 22 verhindert werden kann.

Bei dem Blasenkatheter 10 handelt es sich um einen sogenannten 2-Wege-Katheter. Der Blasenkatheter 10 weist daher an seinem dem Anschlussabschnitt 18 zugewandten Ende einen Verbindungsabschnitt 24 auf. Mit diesem Verbindungsabschnitt 24 ist ein an der Spitze 16 angeordneter, in den Figuren nicht sichtbarer Ballon mit einer Flüssigkeit aufblasbar. Mit Hilfe dieses Ballons kann der Blasenkatheter 10 nach Einführen durch eine Harnröhre in eine Harnblase in der Harnblase befestigt werden. Der Blasenkatheter 10 wird somit durch den Ballon an seiner Position gehalten. Zum Füllen des Ballons ist im Schlauch 12 ein von einem Hauptkanal 28 getrennt vorgesehener Sekundärkanal 30 vorgesehen. Wenn nun am Verbindungsabschnitt 24 Flüssigkeit durch den Sekundärkanal 30 in den Ballon eintreten kann, bläht sich der Ballon auf und hält den Blasenkatheter 10 an seiner Position. Der Hauptkanal 28 wiederum dient zur Verbindung der Spitze 16 des Blasenkatheters 10 mit dem Anschlussabschnitt 18.

An der in Figur 2 dargestellten Spitze 16 kann bei in eine Harnblase eingeführtem Blasenkatheter 10 Urin durch zwei einander gegenüberliegende, augenartige Öffnungen 32 durch den im Einführabschnitt 14 verlaufenden Hauptkanal 28 in am Anschlussabschnitt 18 angeschlossene Anschlusselemente beziehungsweise direkt in eine Toilette abgeleitet werden.

Die in Figur 2 dargestellte Spitze 16 stellt eine Spitze 16 eines Blasenkatheters 10 nach der sogenannte Nelaton-Bauweise dar. Zum leichten Einführen des Blasenkatheters 10 beziehungsweise des Einführabschnitts 14 in eine Harnröhre eines Patienten weist die Spitze 16 daher einen balligen Kegel 34 auf. Dieser ballige Kegel 34 dient dazu, ein leichtes Einführen des Blasenkatheters 10 zu ermöglichen, wobei dies für den Patienten so angenehm wie möglich gestaltet werden soll.

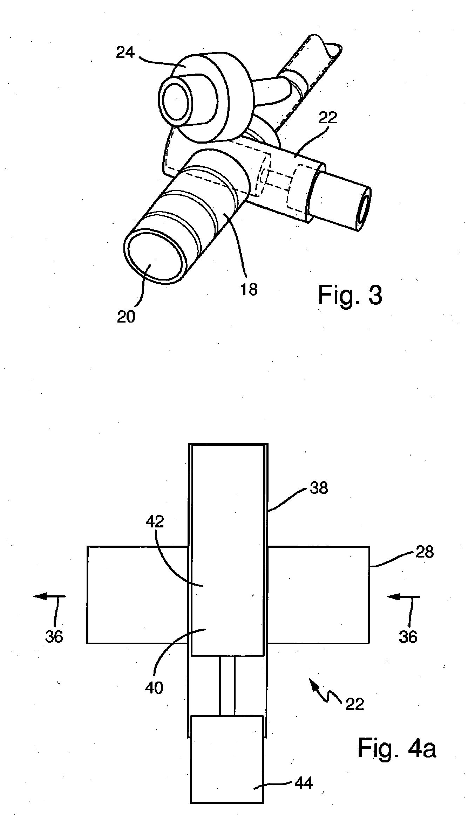

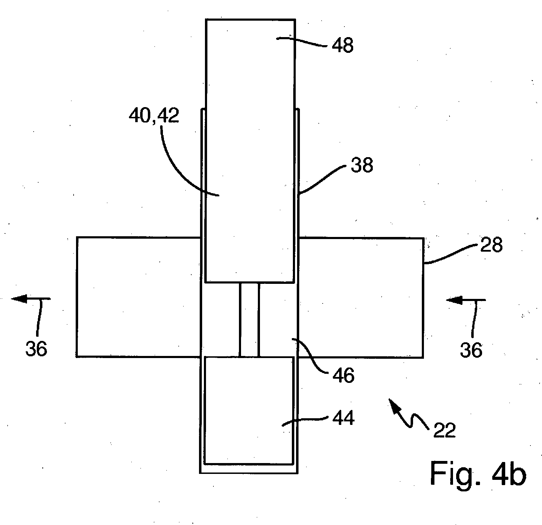

Figur 3 zeigt eine Detailansicht auf den Anschlussabschnitt 18 des Blasenkatheters 10. In die Öffnung 20 am Anschlussabschnitt 18 lassen sich in den Figuren nicht dargestellte Anschlusselemente wie zum Beispiel Urinbeutel anschließen. Der Verbindungsabschnitt 24, welcher mit dem Sekundärkanal 30 zum Füllen des in Figur 3 nicht dargestellten Ballons verbunden ist, ist in Figur 3 deutlich zu erkennen. Ferner ist in Figur 3 das Ventil 22, welches als Schiebeventil ausgebildet ist, in seiner Verschlussstellung dargestellt. Die Funktionsweise des Ventils 22 soll anhand der Figuren 4a und 4b näher beschrieben und erläutert werden.

In Figur 4a ist das Ventil 22 analog zu Figur 3 in seiner Verschlusslage dargestellt. Die Abflussrichtung des Urins durch den Hauptkanal 28 ist durch Pfeile 36 angedeutet. In einem quer zum Hauptkanal verlaufenden Aufnahmeabschnitt 38, welcher als Zylinder ausgebildet ist und vom Hauptkanal 28 durchbrochen wird, ist ein als Kolben ausgebildetes Betätigungselement 40 des Ventils 22 angeordnet. In der in Figur 4a dargestellten Verschlusslage befindet sich ein Verschlussabschnitt 42 des Betätigungselements 40 derart im Hauptkanal 28, dass kein Fluid mehr durch den Hauptkanal 28 abfließen kann.

Beim Druck auf ein unteres Ende 44 des Betätigungselements 40 wird dieses in die in Figur 4b dargestellte Freigabelage bewegt. In dieser Freigabelage kann Fluid beziehungsweise Urin entlang der Pfeile 36 durch den Hauptkanal 28 hindurchfließen. Der Verschlussabschnitt 42 ist dabei im Gegensatz zu Figur 4a aus der Überlagerung mit dem Hauptkanal 28 bewegt. Ein Freigabeabschnitt 46 befindet sich nun in Überlagerung mit dem Hauptkanal 28. Dieser Freigabeabschnitt 46 weist einen deutlich geringeren Außendurchmesser als der Aufnahmeabschnitt 38 auf. Folglich kann Fluid am Freigabeabschnitt 46 vorbei durch den Hauptkanal 28 fließen.

Beim Wechsel eines nicht dargestellten Urinbeutels beziehungsweise nach Entleeren der Blase kann das Ventil 22 des Blasenkatheters 10 wieder geschlossen werden. Dazu muss Druck auf das obere Ende 48 des Betätigungselements 40 ausgeübt werden. Das Betätigungselement 40 wird dabei im Aufnahmeabschnitt 38 wieder in die Verschlusslage bewegt. Folglich kann kein Urin mehr aus dem Blasenkatheter 10. beziehungsweise aus der Öffnung 20 am Anschlussabschnitt 18 austreten.

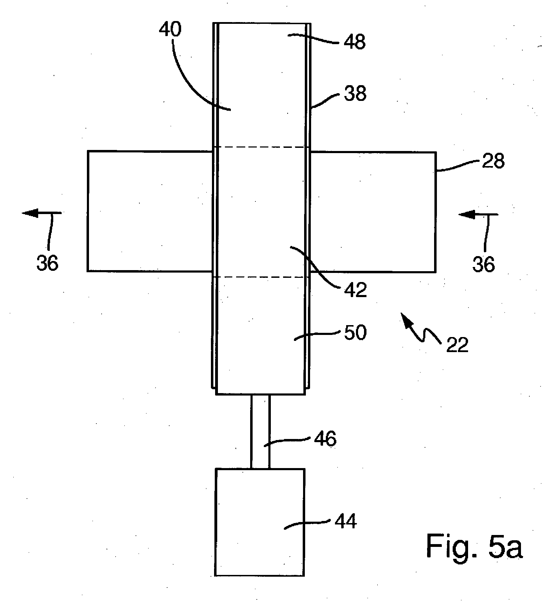

In den Figuren 5a bis 5c ist eine weitere Ausführungsform eines Ventils 22 für einen Blasenkatheter 10 gezeigt. Die den Figuren 4a und 4a entsprechenden Bauteile und Elemente sind mit den entsprechenden Bezugszeichen gekennzeichnet. Das Ventil 22 gemäß den Figuren 5a bis 5c weist drei Schaltstellungen auf, wobei das Ventil 22 in Figur 5a in einer Verschlusslage analog zu Figur 3 und zu Figur 4a geschlossen dargestellt ist. Die Abflussrichtung des Urins durch den Hauptkanal 28 ist ebenfalls durch Pfeile 36 angedeutet. In einem quer zum Hauptkanal 28 verlaufenden Aufnahmeabschnitt 38, welcher als Zylinder ausgebildet ist und vom Hauptkanal 28 durchbrochen wird, ist ein als Kolben ausgebildetes Betätigungselement 40 des Ventils 22 angeordnet.

In der in Figur 5a dargestellten Verschlusslage befindet sich ein Verschlussabschnitt 42 des Betätigungselements 40 derart im Hauptkanal 28, dass kein Fluid mehr durch den Hauptkanal 28 abfließen kann. Unterhalb des Verschlussabschnitts 42 ist ein luftdurchlässiger Filter- und/oder Membranabschnitt 50 angeordnet. Dieser Filter- und/oder Membranabschnitt 50 ist vorzugsweise aus hydrophobem Acrylpolymer hergestellt und ist flüssigkeitsundurchlässig.

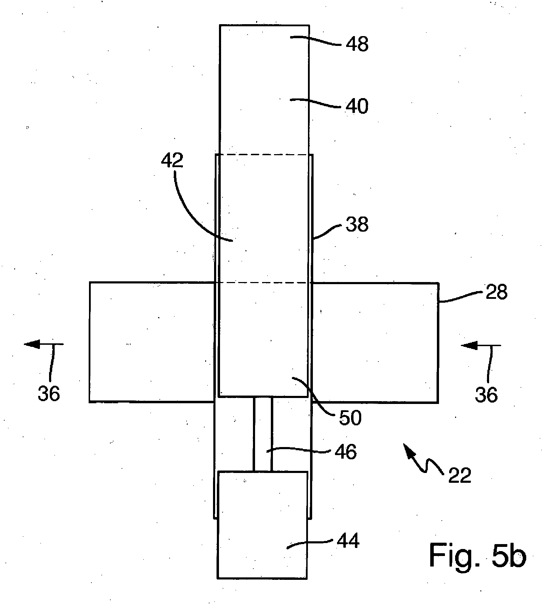

Beim Druck auf ein unteres Ende 44 des Betätigungselements 40 wird dieses zunächst in die in Figur 5b dargestellte Mittelstellung bewegt. In der Mittelstellung befindet sich der Filter- und/oder Membranabschnitt 43 derart in Überlagerung mit dem Hauptkanal 28, dass zwar Luft durch den Hauptkanal 28 austreten kann, wobei jedoch aufgrund des flüssigkeitsundurchlässigen Filter- und/oder Membranabschnitts 50 ein Austreten von Flüssigkeit verhindert werden kann. Folglich kann in der Mittelstellung Luft aus einer Blase eines Patienten entfernt werden.

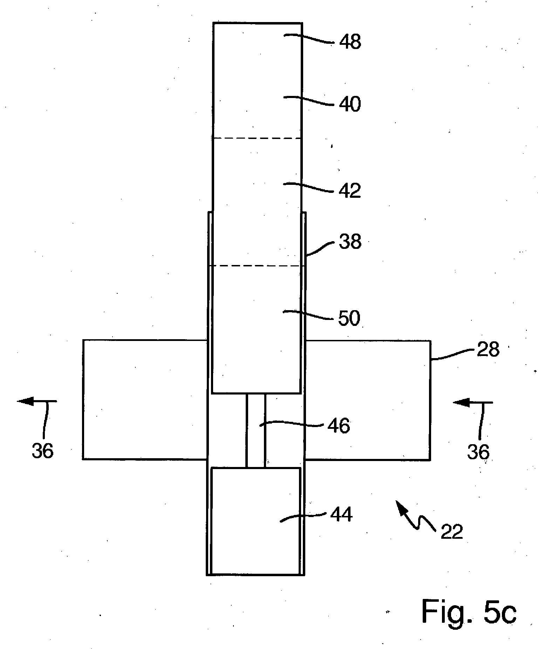

Wenn nun weiter Druck auf das untere Ende 44 des Betätigungselements 40 ausgeübt wird, wird dieses in die in Figur 5c darstellte Freigabelage bewegt. In dieser Freigabelage kann Fluid beziehungsweise Urin entlang der Pfeile 36 durch den Hauptkanal 28 hindurchfließen. Der Verschlussabschnitt 42 ist dabei analog zu Figur 4b aus der Überlagerung mit dem Hauptkanal 28 bewegt. Der Freigabeabschnitt 46 befindet sich nun in Überlagerung mit dem Hauptkanal 28. Dieser Freigabeabschnitt 46 weist einen deutlich geringeren Außendurchmesser als der Aufnahmeabschnitt 38 auf. Folglich kann Fluid am Freigabeabschnitt 46 vorbei durch den Hauptkanal 28 fließen. Ein Wechsel eines Urinbeutels kann analog zum Ventil 22 gemäß Figur 4a und Figur 4b ebenfalls nach Bewegen des Ventils in die Verschlusslage erfolgen. Dazu muss Druck auf das obere Ende 48 des Betätigungselements 40 ausgeübt werden und das Betätigungselement 40 wird im Aufnahmeabschnitt 38 wieder in die Verschlusslage bewegt. Folglich kann auch beim Ventil 22 gemäß der Figuren 5a bis 5c kein Urin mehr aus dem Blasenkatheter 10 beziehungsweise aus der Öffnung 20 am Anschlussabschnitt 18 austreten.

## Patentansprüche

1. Blasenkatheter (10) mit einem Schlauch (12), wobei der Schlauch (12) einen Einführabschnitt (14) zum Einführen über eine Harnröhre in eine Harnblase aufweist und wobei der Schlauch (12) einen Anschlussabschnitt (18) zum Anschließen von Anschlusselementen aufweist, wobei durch den Einführabschnitt (14) Fluid aus der Harnröhre am Anschlussabschnitt (18) ablassbar ist, wobei zwischen dem Einführabschnitt (14) und dem Anschlussabschnitt (18) ein verschließbares Ventil (22) derart angeordnet ist, dass ein Austreten von Fluid aus dem Anschlussabschnitt (18) bei verschlossenem Ventil (22) verhindert werden kann, **dadurch gekennzeichnet, dass** das Ventil (22) als Schiebeventil ausgebildet ist und drei Schaltstellungen aufweist, wobei das Ventil (22) in einer Verschlusslage geschlossen ist und wobei das Ventil (22) in einer Mittelstellung luftdurchlässig ist und wobei das Ventil (22) in einer Freigabelage geöffnet ist.

2. Blasenkatheter (10) nach Anspruch 1, **dadurch gekennzeichnet, dass** im Ventil (22) ein luftdurchlässiger Filter- und/oder Membranabschnitt (50) vorgesehen ist

3. Blasenkatheter (10) nach Anspruch 2, **dadurch gekennzeichnet, dass** der Filter- und/oder Membranabschnitt (50) aus einem hydrophobem Acrylpolymer hergestellt ist.

4. Blasenkatheter (10) nach einem der vorhergehenden Ansprüche, **dadurch gekennzeichnet, dass** der Blasenkatheter (10) an seinem dem Anschlussabschnitt (18) abgewandten Ende eine geschlossene Hohlspitze (16) mit wenigstens zwei gegenüberliegenden Öffnungen (32) nach der Nelaton-Bauweise aufweist.

5. Blasenkatheter (10) nach einem der vorhergehenden Ansprüche, **dadurch gekennzeichnet, dass** der Blasenkatheters (10) ein transurethraler Blasenkatheter (10) ist.

6. Blasenkatheter (10) nach einem der vorhergehenden Ansprüche, **dadurch gekennzeichnet, dass** der Blasenkatheter (10) ein Verweilkatheter ist.

7. Blasenkatheter (10) nach einem der vorhergehenden Ansprüche, **dadurch gekennzeichnet, dass** der Blasenkatheter (10) ein Spülkatheter ist.

## Claims

1. Bladder catheter (10) having a tube (12), the tube (12) having an insertion portion (14) for insertion into a bladder via a urethra and the tube (12) having a connecting portion (18) for connecting-on items for connection, fluid being able to be drained out of the urethra at the connecting portion (18), through the insertion portion (14), and a closable valve (22) being so arranged between the insertion portion (14) and the connecting portion (18) that, with the valve (22) closed, fluid can be prevented from emerging from the connecting portion (18), **characterised in that** the valve (22) takes the form of a slide valve and has three switched positions, the valve (22) being closed in a closed position and the valve (22) being permeable to air in a central position and the valve (22) being open in an open position.

2. Bladder catheter (10) according to claim 1, **characterised in that** a filter and/or membrane portion (50) permeable to air is provided in the valve (22).

3. Bladder catheter (10) according to claim 2, **characterised in that** the filter and/or membrane portion (50) is made of a hydrophobic acrylic polymer.

4. Bladder catheter (10) according to one of the preceding claims, **characterised in that** the bladder catheter (10) has, at its end remote from the connecting portion (18), a closed hollow tip (16) having at least two openings (32) in opposite positions, of the Nelaton type.

5. Bladder catheter (10) according to one of the preceding claims, **characterised in that** the bladder catheter (10) is a transurethral bladder catheter.

6. Bladder catheter (10) according to one of the preceding claims, **characterised in that** the bladder catheter (10) is an indwelling catheter.

7. Bladder catheter (10) according to one of the preceding claims, **characterised in that** the bladder catheter (10) is a lavage catheter.

## Revendications

1. Cathéter urinaire (10) avec une tubulure (12), dans lequel ladite tubulure (12) présente un tronçon d'insertion (14) destiné à être inséré à travers l'urètre dans la vessie, et dans lequel la tubulure (12) comporte un tronçon de raccordement (18) destiné à raccordé à des éléments de raccordement, dans lequel par le tronçon d'insertion (14) du fluide peut être évacué de l'urètre au niveau du tronçon de raccordement (18), **caractérisé en ce qu'**entre le tronçon d'insertion (14) et le tronçon de raccordement (18) est agencée, une valve (22) obturable de telle sorte qu'une fuite de fluide à partir du tronçon de raccordement (18) peut être empêchée par la valve (22) fermée,
**caractérisé en ce que** la valve (22) est formée sous la forme d'une valve à tiroir et présente trois positions de commutation, dans lequel la valve (22) est fermée dans une position de fermeture et dans lequel la valve (22) est perméable à l'air dans une position médiane et dans lequel la valve (22) est ouverte dans une position de libération.

2. Cathéter urinaire (10) selon la revendication 1, **caractérisé en ce qu'**un tronçon de filtre et/ou de membrane (50) perméable à l'air est prévu dans la valve (22).

3. Cathéter urinaire (10) selon la revendication 2, **caractérisé en ce que** le tronçon de filtre et/ou de membrane (50) est constitué d'un polymère acrylique hydrophobe.

4. Cathéter urinaire (10) selon l'une quelconque des revendications précédentes,
**caractérisé en ce que** le cathéter urinaire (10) présente à son extrémité opposée au tronçon de raccordement (18) une pointe creuse fermée (16) avec au moins deux ouvertures opposées (32) d'après la conception Nélaton.

5. Cathéter urinaire (10) selon l'une quelconque des revendications précédentes,
**caractérisé en ce que** le cathéter urinaire (10) est un cathéter urinaire transurétral (10).

6. Cathéter urinaire (10) selon l'une quelconque des revendications précédentes,
**caractérisé en ce que** le cathéter urinaire (10) est un cathéter à demeure.

7. Cathéter urinaire (10) selon l'une quelconque des revendications précédentes,
**caractérisé en ce que** le cathéter urinaire (10) est un cathéter de lavage.
